# FASCICULE DE BREVET EUROPEEN

(11) **EP 1 172 080 B1**
(45) Date de publication et mention de la délivrance du brevet: **14.02.2007**
(21) Numéro de dépôt: 01401578.8
(22) Date de dépôt: 15.06.2001
(51) Int. Cl.: A61Q 5/00, A61K 8/23, A61K 8/27, A61K 8/67

(54) **Utilisation d'un mélange à base de vitamines et de sels minéraux pour diminuer la chute des cheveux et/ou favoriser la repousse des cheveux**
Verwendung einer Zusammensetzung die Vitamine und mineralische Salze enthält zur Verminderung des Haarausfall und/oder zur Förderung des Haarwuchses
Use of a composition comprising vitamins and mineral salts to reduce the hairloss and/or to promote the hairregrowth

(30) Priorité: 11.07.2000 FR 0009063
(43) Date de publication de la demande: 16.01.2002
(73) Titulaire: L'ORÉAL, 75008 Paris (FR)
(72) Inventeur: De Lacharriere, Olivier, 75015 Paris (FR); Pruche, Francis, 60300 Senlis (FR)
(74) Mandataire: Allab, Myriam

(56) Documents cités:
- WO-A-96/10387
- DE-A- 3 800 968
- DE-A- 19 757 921
- DE-A- 19 858 670
- DE-U- 29 812 754
- DE-U- 29 903 490
- FR-A- 2 694 692
- FR-A- 2 723 680
- FR-A- 2 760 358
- FR-A- 2 768 623
- US-A- 4 704 280
- US-A- 6 013 250
- US-A- 6 013 279
- DATABASE CAPLUS [en ligne] retrieved from STN Database accession no. 2000:89325 XP002163780 & JP 2000 038340 A (MITSUYAMA ET AL.) 8 février 2000 (2000-02-08)
- DATABASE WPI Week 199421 Derwent Publications Ltd., London, GB; AN 1994-170436 XP002163781 & HU 209 067 A (TATAR J)

## Description

L'invention est relative à des compositions à base de vitamines et de sels minéraux, destinées à favoriser la repousse des cheveux et/ou diminuer la chute des cheveux et/ou améliorer la qualité des cheveux et/ou favoriser la repigmentation des cheveux.

Chez l'être humain, la croissance des cheveux et leur renouvellement sont principalement déterminés par l'activité des follicules pileux. Leur activité est cyclique et comporte essentiellement trois phases, à savoir la phase anagène, la phase catagène et la phase télogène.

A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150 000 cheveux environ que comporte une chevelure, à chaque instant, 10% d'entre eux environ sont au repos et seront donc remplacés en quelques mois.

Cependant différentes causes peuvent entraîner une perte temporaire ou définitive, des cheveux.
Il peut s'agir de chute et d'altération des cheveux au décours d'une grossesse (post partum), au cours d'états de dénutrition ou de déséquilibres alimentaires, ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut également s'agir d'une alopécie qui est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par atteinte des follicules. Des zones sont touchées préférentiellement, notamment les golfes temporaux ou frontaux chez l'homme, et chez les femmes, on constate une alopécie diffuse du vertex.

Le terme alopécie recouvre aussi toute une famille d'atteintes du follicule pileux ayant pour conséquence finale la perte définitive partielle ou générale des cheveux.
Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

On recherche depuis de nombreuses années, dans l'industrie cosmétique ou pharmaceutique, des composiitons permettant de supprimer ou de réduire l'alopécie, et notamment d'induire ou de stimuler la croissance des cheveux ou de diminuer leur chute.

Dans cette optique, on a certes déjà proposé un grand nombre de compositions comprenant des actifs très divers, comme par exemple le 2,4-diamino 6-piperidinopyrimidine 3-oxyde ou "Minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Le brevet DE19757921 décrit une composition destinée à accélérer la pousse des cheveux, cette composition contenant de la vitamine A, de la vitamine E, de la vitamine C et du zinc. Il ne décrit pas de composition contenant du sélénium.

Le brevet FR760358 décrit une composition destinée à éviter la décoloration capillaire comprenant de la vitamine E, de la vitamine C, du zinc et du sélénium. Il ne décrit pas de composition contenant de vitamine A.

Le brevet DE19858670 décrit une composition destinée à stimuler la pousse des cheveux comprenant des extraits végétaux, de la lécithine, chlorophylle et glycérine, de la silice ou le potassium, de la vitamine A, de la vitamine B2, de la vitamine E, de la vitamine C, de la vitamine

H et du sélénium. Il ne décrit pas de composition contenant du zinc.

Le brevet DE29903490 décrit une composition destinée à la pousse des cheveux qui comprend entre autres de la vitamine A et de la vitamine E. Il ne décrit pas de composition contenant de vitamine C, ni de zinc, ni du sélénium.

Le brevet US6013279 décrit une composition destinée à stimuler la pousse des cheveux, de la peau ou des ongles par la combinaison de vitamines, enzymes et acides aminés. Il ne décrit pas de composition contenant de zinc ni de sélénium.

Le brevet DE29812754 décrit une composition destinée à la pousse des cheveux qui comprend un mélange de vitamines A,B,C,D et K, mais aussi de la biotine, de l'acide folique, du nicotamide. Il ne décrit pas de composition contenant de zinc, ni de sélénium.

Le brevet DE3800968 décrit une composition destinée à régénérer le sang ou la pousse des cheveux. Il ne décrit pas de composition contenant de la vitamine C ni de sélénium.

Le brevet US4704280 décrit une lotion cosmétique applicable sur la peau. Il ne décrit pas une utilisation visant à augmenter le diamètre des cheveux, ni une composition contenant du sélénium.

Le brevet US6013250 décrit une composition destinée à traiter les cheveux contenant des hydrolysats de protéines sulfurées ainsi que des vitamines. Il ne décrit pas de composition contenant du zinc ni de sélénium.

Le brevet JP20000383410 décrit une composition destinée à stimuler la pousse des cheveux qui comprend du minoxidil et des vitamines. Il ne décrit pas de composition contenant de sélénium.

Le brevet FR2694692 décrit une composition cosmétique dermale comprenant des acides gras essentiels, des oligoéléments et des composés vitaminés. Il ne décrit pas une utilisation capillaire.

Le brevet FR2768623 décrit une composition cosmétique pour traiter les peaux grasses. Il ne décrit pas une utilisation capillaire.

Le brevet FR2723680 décrit un complément nutritionnel pour l'équilibre quotidien de l'homme comprenant un mélange complexe de vitamines et de minéraux. Il ne décrit pas d'utilisation capillaire ni de composition contenant de la vitamine A et du sélénium.

Le demande international WO9610387 décrit une composition destinée à stimuler la pousse des cheveux. Elle ne décrit pas de composition contenant de vitamine C ni de sélénium. Le brevet HU209067 décrit une composition destinée à réduire la chute des cheveux contenant un mélange synergique d'herbes, de vitamines et/ou de sels. Il ne décrit pas de composition contenant de vitamine E, de vitamine C, ni du sélénium.

Il reste, d'une manière générale, qu'il serait intéressant et utile de pouvoir disposer de composés actifs autres que ceux déjà connus.

Chez l'homme, il est communément admis depuis des décennies qu'une supplémentation en antioxydants jouait un rôle bénéfique en diminuant l'incidence de certaines affections chroniques telles que les maladies cardiovasculaires et les cancers.
En particulier, des études épidémiologiques et chez l'animal avaient permis de renforcer l'hypothèse que le bêta-carotène jouait un rôle bénéfique dans la prévention de l'apparition de certaines maladies comme celles du cancer du poumon.

Cependant ces résultats ont été controversés suite à la réalisation de deux études de chimio-prévention randomisées, en double aveugle versus placebo dont l'objectif était d'évaluer l'efficacité d'une supplémentation en bêta-carotène (seul ou associé aux vitamines A et E) sur l'incidence de cancers de poumons chez les fumeurs et les travailleurs de l'amiante et/ou de maladies cardiovasculaires.

La première étude, ATBC (Alpha-tocopherol, Beta-carotene Cancer Prevention), réalisée en Finlande a montré une augmentation de 18% des cancers pulmonaires avec 8% de décès supplémentaires parmi les hommes qui ont reçu du bêta-carotène à partir d'une population de 29 139 d'hommes fumeurs.
Aucune réduction sur l'incidence des cancers du poumon après 5 ans de supplémentation n'a été trouvée. (The effect of vitamin E and beta carotene on the incidence of lung cancer and other cancers in male smokers. The alpha-Tocopherol, beta-Carotene Cancer Prevention Study Group. N. Engl. J. Med., 1994; 330 : 1029-35).

La deuxième étude, CARET (Beta-Carotene and Retinol Efficacy Trial), réalisée aux Etats Unis (Seatle), a montré une augmentation de 28% des cancers pulmonaires avec 17% de morts supplémentaires et une augmentation de 26% de morts par maladies cardiovasculaires chez les 18 314 participants (fumeurs et travailleurs exposés à l'amiante) par rapport au groupe placebo. (Omenn GS, Goodman GE, Thorquist MD, et al., Effects of a combination of beta-carotene and vitamin A on lung cancer and cardiovascular disease. N. Engl. J. Med., 1996 ; 334 : 1150-5).

Ces résultats ont confirmé et prolongé les résultats inattendus de l'étude préventive réalisée en Finlande. En effet, loin d'être protectrice comme le laissaient supposer les études épidémiologiques et chez l'animal une supplémentation en bêta-carotène peut s'avérer nuisible.

En conclusion de ces deux essais il a été suggéré que l'effet paradoxal de l'augmentation de la morbidité et de la mortalité observée serait probablement dû aux propriétés du bêta-carotène et sa capacité à générer du stress oxydatif. Ainsi, les agents antioxydants peuvent s'avérer pro-oxydants et conduire à des effets délétères.

II n'y a donc aucune raison pour que l'on imagine utiliser des composés antioxydants dans des compositions, particulièrement des compositions cosmétiques, pour améliorer l'aspect esthétique de la chevelure ou pour lutter contre la chute des cheveux et/ou favoriser leur pousse.

De façon surprenante, la demanderesse a découvert que des compositions à base de vitamines et de sels minéraux, particulièrement des vitamines à effet antioxydant, permettent d'augmenter le diamètre moyen des cheveux, diminuer l'hétérogénéité des diamètres des cheveux (signe associé à l'alopécie), augmenter la densité pilaire, témoignant donc d'une repousse. La demanderesse a de plus constaté que les compositions à base de vitamines et de sels minéraux, particulièrement des vitamines à effet antioxydant, induisent une repigmentation du cheveux, permettant ainsi de lutter contre le blanchiment des cheveux.

Par hétérogénéité des diamètres des cheveux on entend une grande variation des diamètres des cheveux sur une même zone du scalp ; certains cheveux ayant un diamètre physiologique, d'autres ayant, au voisinage immédiat de ces cheveux, un diamètre diminué (cheveux fins). Ainsi par « réduire l'hétérogénéité des diamètres », on entend augmenter le diamètre des cheveux fins.

Par augmenter la densité pilaire on entend augmenter le nombre de cheveux par cm² de cuir chevelu.

Par améliorer la qualité des cheveux, on entend améliorer la coiffabilité et/ou la brillance et/ou l'aspect général de la chevelure.

Ainsi, l'invention a pour objet premier l'utilisation d'une composition à base de vitamines et de sels minéraux destinées à favoriser la repousse des cheveux et/ou freiner la chute des cheveux.

L'invention a pour second objet l'utilisation d'une composition à base de vitamines et de sels minéraux destinées à augmenter le diamètre moyen des cheveux.

L'invention a pour troisième objet l'utilisation d'une composition à base de vitamines et de sels minéraux destinée à diminuer l'hétérogénéité des diamètres des cheveux.

L'invention a pour quatrième objet l'utilisation d'une composition à base de vitamines et de sels minéraux destinée à augmenter la densité pilaire.

L'invention a pour cinquième objet l'utilisation d'une composition à base de vitamines et de sels minéraux destinée à améliorer la qualité et/ou l'aspect de la chevelure.

L'invention a pour sixième objet l'utilisation d'une composition à base de vitamines et de sels minéraux destinée à induire la repigmentation des cheveux.

Ces résultats sont nettement observés pour les femmes ainsi que chez les hommes présentant une alopécie débutante.

L'augmentation du diamètre des cheveux est le paramètre le plus amélioré par un traitement utilisant la composition de l'invention.

Les vitamines de la composition de l'invention sont préférentiellement des vitamines à effet antioxydant choisies parmi la vitamine A (rétinol ou ses esters, ou son équivalent en bêta-carotène), la vitamine E (alpha ou gamma tocophérol), la vitamine C (acide ascorbique ou ses sels) ou les vitamines B.

La composition de l'invention comprend un mélange de vitamine A, vitamine E, et vitamine C.

Les sels minéraux de la composition de l'invention sont préférentiellement choisis parmi le zinc, le sélénium, le fer, le magnésium, le cuivre ou le manganèse.
Selon l'invention, le zinc peut être sous forme de gluconate de zinc, d'oxyde de zinc, de sulfate de zinc ou encore de chlorure de zinc. De même, le fer peut être sous forme de fumarate ferreux, de sulfate ferreux ou encore de chlorure ferreux, le magnésium peut être sous forme d'oxyde de magnésium, le cuivre peut être sous forme d'oxyde de cuivre ou de gluconate de cuivre, le manganèse peut être sous forme de chlorure ou de sulfate de manganèse. Le sélénium peut être sous forme minéral comme le sélénite de sodium, sous forme organique comme la sélénocystéine ou encore sous forme de sélénolevure.

La composition de l'invention comprend un mélange de zinc et de sélénium, par exemple sous forme de sulfate de zinc et de sélénite de sodium.

La composition selon l'invention est composée du mélange de vitamine A, de vitamine E, de vitamine C, de zinc et de sélénium.

La quantité de chacun des éléments de la composition est bien évidemment fonction de l'effet recherché et peut varier dans de large proportion.

Selon l'invention, la composition comprend de la vitamine A en une quantité comprise entre 0,1 mg et 3 mg, de préférence entre 0,5 mg et 1,5 mg, de la vitamine C en une quantité comprise entre 50 mg et 240 mg, de préférence entre 100 mg et 140 mg, de la vitamine E en une quantité comprise entre 10 mg et 60 mg, de préférence entre 40 mg et 50 mg, du zinc en une quantité comprise entre 10 mg et 40 mg, de préférence entre 15 mg et 25 mg et du sélénium en une quantité comprise entre 40 µg et 150 µg, de préférence entre 70 µg et 120 µg.

Lorsque la vitamine A est sous forme d'équivalent bêta-carotène, la composition comprend de 0,6 mg à 18 mg de bêta-carotène et de préférence de 3 mg à 4,5 mg.

Une composition très préférée de l'invention comprend 1 mg de vitamine A (ou 6 mg de bêta-carotène), 120 mg de vitamine C, 30 mg de vitamine E, 20 mg de zinc, et 100 µg de sélénium.

La composition de l'invention peut en outre contenir d'autres actifs anti-oxydants comme des superoxydedismutases (SOD), des catalases, des peroxydases (par exemple la glutathion-péroxydase) et/ou des molécules synthétiques ou des associations possédants des activités enzymatiques mimétiques de ces enzymes (par exemple des complexes de manganèses ou de cuivre avec une activité SOD Like comme le Diisopropylsalicylate de cuivre, Mn(III) tetrakis(4-benzoic acid)porphyrin chloride).

Un autre objet de l'invention est donc l'utilisation d'une composition à base de vitamines et de sels minéraux telle que décrite précédemment comprenant en outre au moins un autre actif anti-oxydant comme une SOD, une catalase, une peroxydase (par exemple la glutathion peroxydase) et/ou une molécule synthétique ou association possédant des activités enzymatiques mimétiques de ces enzymes (par exemple des complexes de manganèses ou de cuivre avec une activité SOD Like comme le Diisopropylsalicylate de cuivre, Mn(lll) tetrakis(4-benzoic acid)porphyrin chloride).

La composition peut en outre comprendre aussi des acides aminés soufrés ou des précurseurs d'acides aminés soufrés, comme par exemple la cystéine, la cystine, la N-acétylcystéine, le glutathion et/ou ses esters, l'oxothiazolidine. De préférence la composition comprend en outre de la L-cystine

La composition de l'invention peut être sous toutes formes galéniques imaginables habituellement utilisées pour le traitement du cuir chevelu, adaptées aussi bien à une application topique sur les cheveux qu'à une administration par la voie orale.

De manière préférentielle, la composition de l'invention est destinée à une administration par la voie orale.

La composition de l'invention est à usage cosmétique.

Très préférentiellement la composition de l'invention est une composition cosmétique destinée à une administration par la voie orale.

La composition de l'invention est une composition cosmétique car elle destinée à améliorer l'aspect général de l'individu qui en fait usage.

Pour une administration par la voie orale, la composition de l'invention peut se présenter sous toutes les formes adaptées, particulièrement sous forme d'une solution buvable, d'un sirop, d'un comprimé, d'une gélule ou encore d'une capsule.
Préférentiellement, la composition de l'invention se présente sous la forme d'une gélule.

L'objet de l'invention est donc l'utilisation d'au moins un mélange de vitamines et de sels minéraux tel que décrit précédemment, dans une composition ou pour la préparation d'un composition destinée à favoriser la repousse des cheveux et/ou diminuer la chute des cheveux et/ou augmenter le diamètre moyen des cheveux et/ou diminuer l'hétérogénéité des diamètres des cheveux et/ou augmenter la densité pilaire et /ou améliorer l'aspect général de la chevelure et/ou favoriser la repigmentation des cheveux.

L'invention a enfin pour objet un procédé de traitement cosmétique des cheveux pour améliorer l'aspect de la chevelure et/ou favoriser la repousse des cheveux et/ou diminuer la chute des cheveux et/ou augmenter le diamètre moyen des cheveux et/ou diminuer l'hétérogénéité des diamètres des cheveux et/ou augmenter la densité pilaire et/ou favoriser la repigmentation des cheveux, consistant à appliquer au moins une des compositions telles que définies précédemment sur le cuir chevelu et/ou les cheveux, puis éventuellement à les rincer à l'eau.

Les exemples qui suivent servent à illustrer l'invention sans toutefois présenter un caractère limitatif.

### Exemple 1 - Capsules molles :

- Huile de Soja 40 mg
- Huile de Germe de Blé 85 mg
- Lécithines de Soja 25 mg
- Vitamine A 1 mg
- Vitamine C 120 mg
- Vitamine E 30 mg
- Zinc 20 mg
- Sélénium 100 µg

### Exemple 2 - Capsules molles :

- Huile de Soja 40 mg
- Huile de Germe de Blé 85 mg
- Lécithines de Soja 25 mg
- Bêta-carotène 6 mg
- Vitamine C 120 mg
- Vitamine E 30 mg
- Zinc 20 mg

### - Sélénium 100 µg

### Exemple 3 - Capsules molles :

- Huile de Soja 40 mg
- Huile de Germe de Blé 85 mg
- Lécithines de Soja 25 mg
- Vitamine A 1 mg
- Vitamine C 120 mg
- Vitamine E 30 mg
- Zinc 20 mg
- Sélénium 100 µg
- Manganèse 5 mg

### Exemple 4 : lotion antichute conditionnée sous forme d'ampoules de 10 ml :

- Vitamine A 1 mg
- Vitamine C 120 mg
- Vitamine E 30 mg
- Zinc 20 mg
- Sélénium 100 µg
solubilisés dans 100 ml d'un mélange de :
- Fructose 0,22 %
- Glucose 0,04 %
- Urée 0,06 %
- NaCl 0,50 %
- Polyéthylèneglycol 0,25 %
- Acide lactique 0,05 %
- Potasse qs pH = 7,5
- Eau/alcool éthylique à 31 % en poids qsp 100,00 %

### Exemple 4 : lotion antichute conditionnée sous forme d'ampoules de 10 ml :

- Bêta-carotène 6 mg
- Vitamine C 120 mg
- Vitamine E 30 mg
- Zinc 20 mg
- Sélénium 100 µg
solubilisés dans 100 ml d'un mélange de :
- Fructose 0,22 %
- Glucose 0,04 %
- Urée 0,06 %
- NaCl 0,50 %
- Polyéthylèneglycol 0,25 %
- Acide lactique 0,05 %
- Potasse qs pH = 7,5
- Eau/alcool éthylique à 31 % en poids qsp 100,00 %

## Revendications

1. Utilisation d'un mélange de vitam ne A, de vitamine E, de vitamine C, de zinc et de sélénium dans une composition, comme agent pour augmenter le diamètre moyen des cheveux, diminuer l'hétérogénéité des diamètres des cheveux, et/ou augmenter la densité pilaire.

2. Utilisation d'un mélange selon la revendication 1 comme agent pour améliorer la qualité et/ou l'aspect de la chevelure.

3. Utilisation d'un mélange selon l'une quelconque des revendications 1 ou 2, **caractérisée par le fait que** la vitamine A est en une quantité comprise entre 0,1 mg et 3 mg.

4. Utilisation d'un mélange selon la revendication 3, **caractérisée par le fait que** la vitamine A est en une quantité comprise entre 0,5 mg et 1,5 mg.

5. Utilisation d'un mélange selon l'une quelconque des revendications 1 à 4, **caractérisée par le fait que** la vitamine C est en une quantité comprise entre 50 mg et 240 mg.

6. Utilisation d'un mélange selon la revendication 5, **caractérisée par le fait que** la vitamine C est en une quantité comp ise entre 100 mg et 140 mg.

7. Utilisation d'un mélange selon l'une quelconque des revendications 1 à 6, **caractérisée par le fait que** la vitamine E est en une quantité comprise entre 10 mg et 60 mg.

8. Utilisation d'un mélange selon la revendication 7. **caractérisée par le fait que** la vitamine E est en une quantité comprise entre 40 mg et 50 mg.

9. Utilisation d'un mélange selon l'une quelconque des revendications 1 à 8, **caractérisée par le fait que** le zinc est en une quantité comprise entre 10 mg et 40 mg.

10. Utilisation d'un mélange selon la revendication 9, **caractérisée par le fait que** le zinc est en une quantité comprise entre 15 mg et 25 mg.

11. Utilisation d'un mélange selon l'une quelconque des revendications 1 à 10, **caractérisée par le fait que** le sélénium est en une quantité comprise entre 40 µg et 150 µg.

12. Utilisation d'un mélange selon la revendication 11, **caractérisée par le fait que** le sélénium est en une quantité comprise entre 70 µg et 120 µg.

13. Utilisation d'un mélange selon l'une quelconque des revendications 1 à 12, **caractérisée par le fait que** la composition est destinée à une administration par la voie orale.

14. Utilisation d'un mélange selon l'une quelconque des revendications 1 à 13, **caractérisée par le fait que** la composition est destinée à une application cosmétique.

15. Utilisation d'un mélange selon l'une quelconque des revendications 1 à 14, **caractérisée par le fait que** la composition contient au moins des sels minéraux choisis parmi le zinc, le sélénium le fer, le magnésium, le cuivre ou le manganèse.

16. Utilisation d'un mélange selon l'une quelconque des revendications 1 à 15, **caractérisée par le fait que** la composition contient des vitamines choisies parmi la vitamine A, la vitamine E, la vitamine C ou les vitamines B.

17. Utilisation d'un mélange selon l'une quelconque des revendications 1 à 16, **caractérisée par le fait que** la composition contient en outre un autre actif anti-oxydant comme une SOD, une catalase, une peroxydase (par exemple la glutathion-péroxydase) et/ou une molécule synthétique ou association possédant des activités enzymatiques mimétiques de ces enzymes (par exemple des complexes de manganèses ou de cuivre avec une activité SOD Like comme le Diisopropylsalicylate de cuivre, Mn(II) tetrakis(4-benzoic acid)porphyrin chloride) ou encore un acide aminé soufré.

18. Composition contenant un agent pour augmenter le diamètre moyen des cheveux, diminuer l'hétérogénéité des diamètres des cheveux, et/ou augmenter la densité pilaire selon l'une que conque des revendications précédentes, **caractérisée par le fait qu'**elle comprend 1 mg de vitamine A, 120 mg de vitamine C, 30 mg de vitamine E, 20 mg de zinc, et 100 µg de sélénium.

19. Composition selon la revendication 18, **caractérisée par le fait que** la vitamine A est sous forme d'équivalent bêta-carotène.

20. Composition selon la revendication précédente, **caractérisée par le fait que** le bêta-carotène est en une quantité comprise entre 0,6 mg et 18 mg.

21. Composition selon la revendication précédente, **caractérisée par le fait que** le bêta-carotène est en une quantité comprise entre 3 mg à 4,5 mg.

22. Composition selon l'une quelconque des revendications 19 à 21, **caractérisée par le fait qu'**elle comprend 6 mg de bêta-carotène, 120 mg de vitamine C, 30 mg de vitamine E, 20 mg de zinc, et 100 µg de sélénium,

23. Utilisation d'une composition à base de vitamines et de sels minéraux telle que décrite dans l'une quelconque des revendications 1 à 22, pour augmenter le diamètre moyen des cheveux et/ou pour diminuer l'hétérogénéité des diamètres des cheveux et/ou pour augmenter la densité pilaire et/ou pour améliorer la qualité et/ou l'aspect de la chevelure.

24. Procédé de traitement cosmétique e des cheveux pour augmenter le diamètre moyen des cheveux et/ou pour diminuer l'hétérogénéité des diamètres des cheveux et/ou pour augmenter la densité pilaire et/ou pour améliorer la qualité et/ou l'aspect de la chevelure, consistant à appliquer sur le cuir chevelu et/ou les cheveux au moins une composition elle que définie dans l'une quelconque des revendications 1 à 22. puis éventuellement a les rincer à l'eau.

## Claims

1. Use of a mixture of vitamin A, vitamin E, vitamin C, zinc and selenium in a composition, as an agent for increasing the mean diameter of hair, decreasing the heterogeneity of the diameter of hair and/or increasing hair density.

2. Use of a mixture according to Claim 1, as an agent for improving the quality and/or the appearance of the head of hair.

3. Use of a mixture according to either of Claims 1 and 2, **characterized in that** the vitamin A is in an amount of between 0.1 mg and 3 mg.

4. Use of a mixture according to Claim 3, **characterized in that** the vitamin A is in an amount of between 0.5 mg and 1.5 mg.

5. Use of a mixture according to any one of Claims 1 to 4, **characterized in that** the vitamin C is in an amount of between 50 mg and 240 mg.

6. Use of a mixture according to Claim 5, **characterized in that** the vitamin C is in an amount of between 100 mg and 140 mg.

7. Use of a mixture according to any one of Claims 1 to 6, **characterized in that** the vitamin E is in an amount of between 10 mg and 60 mg.

8. Use of a mixture according to Claim 7, **characterized in that** the vitamin E is in an amount of between 40 mg and 50 mg.

9. Use of a mixture according to any one of Claims 1 to 8, **characterized in that** the zinc is in an amount of between 10 mg and 40 mg.

10. Use of a mixture according to Claim 9, **characterized in that** the zinc is in an amount of between 15 mg and 25 mg.

11. Use of a mixture according to any one of Claims 1 to 10, **characterized in that** the selenium is in an amount of between 40 µg and 150 µg.

12. Use of a mixture according to Claim 11, **characterized in that** the selenium is in an amount of between 70 µg and 120 µg.

13. Use of a mixture according to any one of Claims 1 to 12, **characterized in that** the composition is intended for oral administration.

14. Use of a mixture according to any one of Claims 1 to 13, **characterized in that** the composition is intended for a cosmetic application.

15. Use of a mixture according to any one of Claims 1 to 14, **characterized in that** the composition contains at least inorganic salts chosen from zinc, selenium, iron, magnesium, copper and manganese.

16. Use of a mixture according to any one of Claims 1 to 15, **characterized in that** the composition contains vitamins chosen from vitamin A, vitamin E, vitamin C and B vitamins.

17. Use of a mixture according to any one of Claims 1 to 16, **characterized in that** the composition also contains another antioxidant active agent, such as an SOD, a catalase, a peroxidase (for example glutathione peroxidase) and/or a synthetic molecule or association having enzymatic activities which mimic these enzymes (for example manganese or copper complexes with an SOD-like activity, such as copper diisopropylsalicylate or Mn(III) tetrakis(4-benzoic acid)porphyrin chloride), or a sulphur-containing amino acid.

18. Composition containing an agent for increasing the mean diameter of hair, decreasing the heterogeneity of the diameter of hair and/or increasing hair density according to any one of the preceding claims, **characterized in that** it comprises 1 mg of vitamin A, 120 mg of vitamin C, 30 mg of vitamin E, 20 mg of zinc and 100 µg of selenium.

19. Composition according to Claim 18, **characterized in that** the vitamin A is in the form of beta-carotene equivalent.

20. Composition according to the preceding claim, **characterized in that** the beta-carotene is in an amount of between 0.6 mg and 18 mg.

21. Composition according to the preceding claim, **characterized in that** the beta-carotene is in an amount of between 3 mg and 4.5 mg.

22. Composition according to any one of Claims 19 to 21, **characterized in that** it comprises 6 mg of beta-carotene, 120 mg of vitamin C, 30 mg of vitamin E, 20 mg of zinc and 100 µg of selenium.

23. Use of a composition based on vitamins and on inorganic salts, as described in any one of Claims 1 to 22, for increasing the mean diameter of hair and/or for decreasing the heterogeneity of the diameter of hair and/or for increasing hair density and/or for improving the quality and/or the appearance of the head of hair.

24. Cosmetic hair treatment process for increasing the mean diameter of hair and/or for decreasing the heterogeneity of the diameter of hair and/or for increasing hair density and/or for improving the quality and/or the appearance of the head of hair, which consists in applying at least one composition as defined in any one of Claims 1 to 22 to the scalp and/or the hair, and then, optionally, in rinsing it with water.

## Patentansprüche

1. Verwendung eines Gemischs von Vitamin A, Vitamin E, Vitamin C, Zink und Selen in einer Zusammensetzung als Mittel zur Vergrößerung des mittleren Durchmessers der Haare, zur Verringerung der Heterogenität der Durchmesser der Haare und/oder zur Erhöhung der Haardichte.

2. Verwendung eines Gemischs nach Anspruch 1 als Mittel zur Verbesserung der Qualität und/oder des Aussehens des Haars.

3. Verwendung eines Gemischs nach Anspruch 1 oder 2, **dadurch gekennzeichnet, dass** das Vitamin A in einer Menge von 0,1 bis 3 mg vorliegt.

4. Verwendung eines Gemischs nach Anspruch 3, **dadurch gekennzeichnet, dass** das Vitamin A in einer Menge von 0,5 bis 1,5 mg vorliegt.

5. Verwendung eines Gemischs nach einem der Ansprüche 1 bis 4, **dadurch gekennzeichnet, dass** das Vitamin E in einer Menge von 50 bis 240 mg vorliegt.

6. Verwendung eines Gemischs nach Anspruch 5, **dadurch gekennzeichnet, dass** das Vitamin C in einer Menge von 100 bis 1450 mg vorliegt.

7. Verwendung eines Gemischs nach einem der Ansprüche 1 bis 6, **dadurch gekennzeichnet, dass** das Vitamin E in einer Menge von 10 bis 60 mg vorliegt.

8. Verwendung eines Gemischs nach Anspruch 7, **dadurch gekennzeichnet, dass** das Vitamin E in einer Menge von 40 bis 50 mg vorliegt.

9. Verwendung eines Gemischs nach einem der Ansprüche 1 bis 8, **dadurch gekennzeichnet, dass** das Zink in einer Menge von 10 bis 40 mg vorliegt.

10. Verwendung eines Gemischs nach Anspruch 9, **dadurch gekennzeichnet, dass** das Zink in einer Menge von 15 bis 25 mg vorliegt.

11. Verwendung eines Gemischs nach einem der Ansprüche 1 bis 10, **dadurch gekennzeichnet, dass** das Selen in einer Menge von 40 bis 150 µg vorliegt.

12. Verwendung eines Gemischs nach Anspruch 11, **dadurch gekennzeichnet, dass** das Selen in einer Menge von 70 bis 120 µg vorliegt.

13. Verwendung eines Gemischs nach einem der Ansprüche 1 bis 12, **dadurch gekennzeichnet, dass** die Zusammensetzung für eine orale Verabreichung bestimmt ist.

14. Verwendung nach einem der Ansprüche 1 bis 13, **dadurch gekennzeichnet, dass** die Zusammensetzung für eine kosmetische Anwendung bestimmt ist.

15. Verwendung eines Gemischs nach einem der Ansprüche 1 bis 14, **dadurch gekennzeichnet, dass** die Zusammensetzung zumindest anorganische Salze enthält, die unter Zink, Selen, Eisen, Magnesium, Kupfer oder Mangan ausgewählt sind.

16. Verwendung eines Gemischs nach einem der Ansprüche 1 bis 15, **dadurch gekennzeichnet, dass** die Zusammensetzung Vitamine enthält, die ausgewählt sind unter Vitamin A, Vitamin E, Vitamin C oder den Vitaminen B.

17. Verwendung eines Gemischs nach einem der Ansprüche 1 bis 16, **dadurch gekennzeichnet, dass** die Zusammensetzung ferner enthält: einen weiteren antioxidativen Wirkstoff wie ein SOD, eine Catalase, eine Peroxidase (zum Beispiel Glutathionperoxidase) und/oder ein synthetisches Molekül oder eine Kombination mit enzymatischen Aktivitäten, welche diese Enzyme nachbilden (zum Beispiel Komplexe von Mangan oder Kupfer mit einer SOD-artigen Aktivität, wie Kupfer-Diisopropylsalicylat, Mn(II)-Tetrakis(4-benzoesäure)-porphyrinchlorid) oder auch eine schwefelhaltige Aminosäure.

18. Zusammensetzung, die ein Mittel zur Vergrößerung des mittleren Durchmessers der Haare, zur Verringerung der Heterogenität der Durchmesser der Haare und/oder zur Erhöhung der Haardichte nach einem der vorhergehenden Ansprüche enthält, **dadurch gekennzeichnet, dass** sie 1 mg Vitamin A, 120 mg Vitamin C, 30 mg Vitamin E, 20 mg Zink und 100 µg Selen enthält.

19. Zusammensetzung nach Anspruch 18, **dadurch gekennzeichnet, dass** das Vitamin A in Form eines Äquivalents β-Carotin vorliegt.

20. Zusammensetzung nach dem vorhergehenden Anspruch, **dadurch gekennzeichnet, dass** das β-Carotin in einer Menge von 0,6 bis 18 mg vorliegt.

21. Zusammensetzung nach dem vorhergehenden Anspruch, **dadurch gekennzeichnet, dass** das β-Carotin in einer Menge von 3 bis 4,5 mg vorliegt.

22. Zusammensetzung nach einem der Ansprüche 19 bis 21, **dadurch gekennzeichnet, dass** sie 6 mg β-Carotin, 120 mg Vitamin C, 30 mg Vitamin E, 20 mg Zink und 100 µg Selen enthält.

23. Verwendung einer Zusammensetzung auf der Basis von Vitaminen und Mineralsalzen wie in einem der Ansprüche 1 bis 22 beschrieben zur Vergrößerung des mittleren Durchmessers der Haare und/oder zur Verringerung der Heterogenität der Durchmesser der Haare und/oder zur Erhöhung der Haardichte und/oder zur Verbesserung der Qualität und/oder des Aussehens des Haars.

24. Verfahren zur kosmetischen Behandlung der Haare zur Vergrößerung des mittleren Durchmessers der Haare und/oder zur Verringerung der Heterogenität der Durchmesser der Haare und/oder zur Erhöhung der Haardichte und/oder zur Verbesserung der Qualität und/oder des Aussehens des Haars, das darin besteht, dass mindestens eine Zusammensetzung wie in einem der Ansprüche 1 bis 22 definiert auf die Kopfhaut und/oder die Haare aufgebracht wird, worauf diese gegebenenfalls mit Wasser gespült werden.
